# EUROPÄISCHE PATENTSCHRIFT

(11) **EP 0 338 493 B1**
(45) Veröffentlichungstag und Bekanntmachung des Hinweises auf die Patenterteilung: **16.10.1996**
(21) Anmeldenummer: 89106895.9
(22) Anmeldetag: 18.04.1989
(51) Int. Cl.: C07D 333/38, C07D 333/32, C07D 333/36

(54) **Tetrahydro-4-phenylimino-3-thiophenessigsäuren**
Tetrahydro-4-phenylimino-3-thiophen-acetic acids
Acides tétrahydro-4-phénylimino-3-thiophène-acétiques

(30) Priorität: 22.04.1988 DE 3813643
(43) Veröffentlichungstag der Anmeldung: 25.10.1989
(73) Patentinhaber: Byk Gulden Lomberg Chemische Fabrik GmbH, D-78403 Konstanz (DE)
(72) Erfinder: Figala, Volker, Dr., D-7753 Allensbach 4 (DE)

(56) Entgegenhaltungen:
- EP-A- 0 005 559
- EP-A- 0 210 320

## Beschreibung

### Anwendungsgebiet der Erfindung

Die Erfindung betrifft neue Tetrahydro-4-phenylimino-3-thiophenessigsäuren, Verfahren zu ihrer Herstellung und ihre Verwendung zur Synthese von pharmazeutischen Wirkstoffen, die in der pharmazeutischen industrie zur Herstellung von Arzneimitteln eingesetzt werden.

### Bekannter technischer Hintergrund

Im europäischen Patent Nr. 5559 wird die Herstellung bestimmter Phenylaminothiophenessigsäurederivate beschrieben, die als Wirkstoffe in Arzneimitteln Verwendung finden sollen. In der europäischen Patentanmeldung Nr. 210 320 werden Tetrahydrothien-3-ylidenimine offenbart und es wird die Dehydrierung dieser Verbindungen zu den entsprechenden N-Thienylacetamiden beschrieben.

### Beschreibung der Erfindung

Gegenstand der Erfindung sind sowohl ein neues Verfahren zur Herstellung bestimmter Phenylaminothiophenessigsäurederivate als auch neue Ausgangsprodukte, die in diesem Verfahren eingesetzt werden. Die Erfindung beruht auf der überraschenden Tatsache, daß sich die neuen Ausgangsprodukte nicht in der aus der europäischen Patentanmeldung Nr. 210 320 bekannten Weise mittels Thionylchlorid oxydieren (dehydrieren) lassen, sondern daß vielmehr eine überraschend glatte und mit hohen Ausbeuten verbundene Oxydation mit Hilfe von Brom gelingt.

Ein erster Aspekt der Erfindung sind somit neue Iminoverbindungen der Formel I 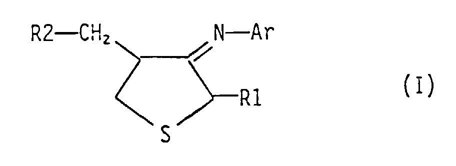 worin
- R1: Wasserstoff oder -CO-O-R6 bedeutet,
- R2: -CO-O-R7 bedeutet,
- Ar: 2,6-Dichlorphenyl bedeutet,
- R6: Wasserstoff, Methyl oder Ethyl bedeutet und
- R7: Wasserstoff, Methyl, Ethyl oder 2-Hydroxy-ethoxy-ethyl bedeutet, und ihre Salze.

Als Salze kommen alle herstellbaren Salze, insbesondere die Salze der Säuren in Frage. Hier seien beispielsweise die Alkalimetall- (z.B. Natrium-, Kalium-), Erdalkalimetall- (z.B. Calcium-, Magnesium-) oder Erdmetall- (z.B. Aluminium-) -salze genannt.

Die Verbindungen der Formel I und ihre Salze sind wertvolle Ausgangsprodukte zur Herstellung der im europäischen Patent 5559 beschriebenen Phenylaminothiophenessigsäurederivate der Formel II, 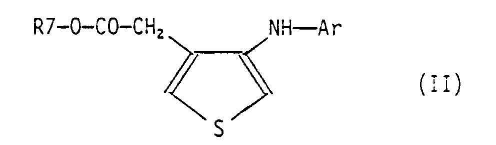 worin Ar und R7 die obengenannten Bedeutungen haben, sowie von weiteren - zur Herstellung der Phenylaminothiophenessigsäurederivate der Formel II benötigten - Zwischenprodukten der Formel III, 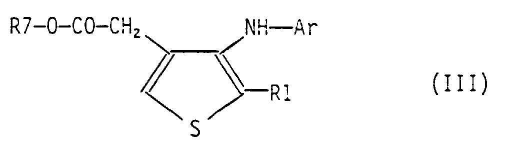 worin R1 -CO-O-R6 bedeutet und Ar, R6 und R7 die obengenannten Bedeutungen haben.

Die Erfindung betrifft somit in einem weiteren Aspekt die Verwendung der Verbindungen der Formel I in einem Verfahren zur Herstellung von Verbindungen der Formel IV, 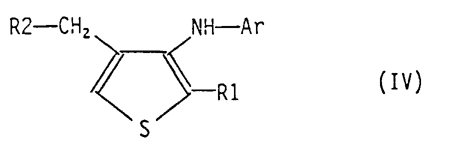 worin R1 Wasserstoff bedeutet und R2 und Ar die obengenannten Bedeutungen haben, und ihren Salzen.

Das Verfahren ist dadurch gekennzeichnet, daß man Verbindungen der Formel I oder ihre Salze mit Brom dehydriert (oxidiert).

Die Dehydrierung (Oxidation) der Verbindungen der Formel I kann in zweckmäßigen, inerten Lösungsmitteln durchgeführt werden. Als Lösungsmittel eignen sich beispielsweise Ether, wie Dioxan, Diethylether, Tetrahydrofuran, Glykolmonoethylether oder Glykoldimethylether; Alkohole, wie Ethanol oder Methanol; aromatische Kohlenwasserstoffe, wie Xylol oder Toluol; oder chlorierte Kohlenwasserstoffe, wie Methylenchlorid, Chloroform, Tetrachlorethan oder Dichlorethan; oder polare aprotische Lösungsmittel, wie Dimethylformamid oder Dimethylsulfoxid.

Die Reaktionstemperaturen können in einem weiten Bereich variieren. Im allgemeinen wird die Umsetzung bei Temperaturen zwischen -70°C und 80°C, vorzugsweise zwischen -20°C und +20°C, insbesondere bei Temperaturen um oder unterhalb 0°C durchgeführt.

Die Isolierung und Reinigung der erhaltenen Verbindungen IV erfolgt in an sich bekannter Weise z.B. derart, daß man das Lösungsmittel, gewünschtenfalls nach vorheriger Freisetzung der Verbindung IV aus ihren Salzen, z.B. durch Versetzen mit Wasser und Abtrennen der wäßrigen Phase, im Vakuum abdestilliert und den erhaltenen Rückstand aus einem geeigneten Lösungsmittel umkristallisiert oder einer der üblichen Reinigungsmethoden unterwirft.

Weiterer Gegenstand der Erfindung ist ein Verfahren zur Herstellung der Verbindungen der Formel I und ihrer Salze. Das Verfahren ist dadurch gekennzeichnet, daß man Ketone der Formel V oder ihre Salze, 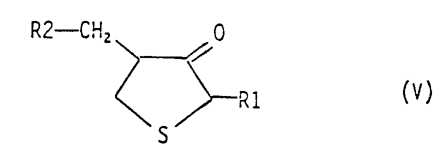 worin R1 und R2 die oben angegebenen Bedeutungen haben, in Form ihrer Ketale mit 2,6-Dichloranilin umsetzt und gewünschtenfalls anschließend erhaltene Salze in die freien Verbindungen oder erhaltene freie Verbindungen in die Salze überführt.

Die Umsetzung der Ketale mit 2,6-Dichloranilin wird auf eine Weise vorgenommen, wie sie für die Herstellung von Anilinen dem Fachmann vertraut ist. Die Umsetzung erfolgt ohne Lösungsmittel oder in einem inerten, vorzugsweise höhersiedenden Lösungsmittel, beispielsweise in einem Ether, wie Dioxan, Glykolmonoethylether oder Glykoldimethylether, oder in einem aromatischen Kohlenwasserstoff, wie Xylol oder Toluol.

Die Reaktionstemperaturen liegen im allgemeinen zwischen 50°C und 200°C, insbesondere bei der Siedetemperatur des verwendeten Lösungsmittels oder - wenn ohne Lösungsmittel gearbeitet wird - zwischen 120°C und 160°C.

Die Ketone V werden hergestellt durch Umsetzung von Itaconsäurederivaten VI, 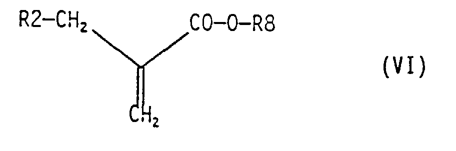 worin R2 -CO-O-R7 bedeutet, und R7 und R8 vorzugsweise Methyl oder Ethyl bedeuten, mit Thioglykolsäureestern VII,

HS-CH₂-R1 (VII)

worin R1 -CO-O-R6 und R6 Methyl oder Ethyl bedeutet, in Gegenwart einer Base bzw. nach Deprotonierung des Thioglykolsäureesters mit einer Base.

Die Umsetzung von VI mit VII erfolgt auf eine dem Fachmann geläufige Weise, beispielsweise so, wie sie nachfolgend in den Beispielen beschrieben ist. Die Herstellung der Ketale der Ketone V erfolgt ebenfalls auf eine dem Fachmann vertraute Weise, beispielsweise durch Umsetzung der Ketone V mit Orthoameisensäureestern.

Die Ketone V, in denen R1 Wasserstoff bedeutet, werden aus den (durch Umsetzung von VI mit VII ursprünglich entstandenen) Ketonen V, worin R1 -CO-O-R6 bedeutet, durch Decarboxylierung bzw. durch Verseifung und anschließende Decarboxylierung erhalten. Die Decarboxylierung erfolgt durch Erhitzen, beispielsweise auf Temperaturen zwischen 50°C und 100°C, in saurem Medium, beispielsweise in 2N Salzsäure.

Die folgenden Beispiele erläutern die Erfindung näher. Fp. steht für Schmelzpunkt.

### Beispiele

### 1. 4-(2,6-Dichloranilino)-3-thiophenessigsäuremethylester

Zu einer Lösung von 318 g (1 Mol) Tetrahydro-4-(2,6-dichlorphenylimino)-3-thiophenessigsäuremethylester in 1 l Dichlormethan wird bei -15°C eine Lösung von 51,23 ml Brom in 75 ml Dichlormethan zugetropft. Anschließend wird in die entstandene Suspension eine Lösung von 50 g Natriumsulfit in 400 ml Wasser eingerührt. Die organische Phase wird abgetrennt, die wässrige Phase noch zweimal mit Dichlormethan ausgeschüttelt. Die vereinigten organischen Phasen werden getrocknet und eingeengt. Umkristallisation aus Methanol liefert die reine Titelverbindung vom Fp.: 112-113°C.

Der Tetrahydro-4-(2,6-dichlorphenylimino)-3-thiophenessigsäuremethylester wird auf folgendem Weg erhalten:

160 g Tetrahydro-4-oxo-3-thiophenessigsäure werden in einer Mischung von 212 ml Trimethylorthoformiat, 123 ml absolutem Methanol, 37 ml Toluol und 1,9 ml konz. Schwefelsäure suspendiert und gerührt. Nach 1 h entsteht eine Lösung, die 16 h bei Raumtemperatur stehengelassen wird. Bei 40°C wird anschließend überschüssiger Orthoester und Lösungsmittel unter Feuchtigkeitsausschluß im Wasserstrahlvakuum abdestilliert. Zu dem öligen Rückstand von Tetrahydro-4,4-dimethoxy-3-thiophenessigsäuremethylester werden 162,2 g 2,6-Dichloranilin zugefügt und auf 140°C erwärmt. Entstehendes Methanol wird abdestilliert. Nach dem Abkühlen erhält man 4-(2,6-Dichlorphenylimino)-3-thiophenessigsäuremethylester (R_{f} = 0,61, Kieselgel; Essigester/Dichlormethan 1:9) als Öl, das ohne weitere Reinigung weiterverarbeitet wird.

Die Tetrahydro-4-oxo-3-thiophenessigsäure wird auf folgendem Weg hergestellt:

Zu 380 ml einer 5,25 M Natriummethylatlösung in Methanol werden 178,6 ml Thioglykolsäuremethylester bei 10°C zugetropft. Anschließend werden bei der gleichen Temperatur 310,8 g Itaconsäuremethylester zugetropft. Das auf ein Volumen von etwa 750 ml eingeengte Reaktionsgemisch wird unter gutem Rühren zu einer Lösung von 240 g CaCl₂ in 450 ml Wasser zugetropft. Das ausgefallene Calcium-di-(4,5-dihydro-2-methoxycarbonyl-4-methoxycarbonylmethyl-3-thienyl-oxid) wird abgesaugt, mit wenig eiskaltem Wasser gewaschen und in 1426 ml auf 70°C erwärmte 2N Salzsäure eingetragen. Nachdem etwa 1 l Methanol mit Wasser abdestilliert wurde, wird abgekühlt und das ausgefallene Produkt abgesaugt. Umkristallisation aus Toluol liefert Tetrahydro-4-oxo-3-thiophenessigsäure vom Fp. 87-89°C.

### 2. 4-(2,6-Dichloranilino)-3-thiophenessigsäure

a) 3,18 g (10 mMol) 4-(2,6-Dichlorphenylimino)-tetrahydro-3-thiophenessigsäuremethylester werden zu einer Lösung von 560 mg (10 mMol) Kalilauge in 5 ml Methanol und 5 ml Wasser gegeben. Es wird 0,5 h auf 80°C erhitzt, abgekühlt und mit 200 ml Dichlormethan versetzt. Durch Einengen auf ein Volumen von 20 ml wird Wasser und Methanol mit überschüssigem Dichlormethan azeotrop entfernt. Unter Feuchtigkeitsausschluß wird die Reaktionslösung auf -20°C abgekühlt und tropfenweise mit 1,58 g (10 mMol) Brom in 5 ml Dichlormethan versetzt. Das Reaktionsgemisch wird auf Raumtemperatur erwärmt, mit natriumsulfithaltiger 2N Natronlauge extrahiert, die wässrige Phase wird mit 2N Salzsäure auf pH 2 gestellt und mit Dichlormethan ausgeschüttelt. Die organische Phase wird getrocknet und zur Trockene eingeengt. Umkristallisation des Rückstands aus Toluol liefert die Titelverbindung vom Fp. 178°C.
b) Zu einer Suspension von 6,74 g (10 mMol) Calcium-di-[4-(2,6-dichlorphenylimino)]-tetrahydro-3-thiophenacetat in 40 ml Dichlormethan werden bei -20°C 3,16 g (20 mMol) Brom in 6 ml Dichlormethan zugetropft. Das Reaktionsgemisch wird auf Raumtemperatur erwärmt und mit natriumsulfithaltiger 2N Natronlauge extrahiert. Die wässrige Phase wird mit 2N Schwefelsäure auf pH 2 gebracht und mit Dichlormethan ausgeschüttelt. Die organische Phase wird getrocknet und zur Trockene eingeengt. Umkristallisation des Rückstands aus Toluol liefert 4-(2,6-Dichloranilino)-3-thiophenessigsäure.

Das Calcium-di-[4-(2,6-dichlorphenylimino)]-tetrahydro-3-thiophenacetat wird auf folgendem Weg erhalten:

3,18 g (10 mMol) 4-(2,6-Dichlorphenylimino)-tetrahydro-3-thiophenessigsäuremethylester werden zu einer Lösung von 560 mg (10 mMol) Kalilauge in 5 ml Methanol und 5 ml Wasser gegeben. Es wird 0,5 h auf 80°C erhitzt und anschließend abgekühlt. Das Reaktionsgemisch wird zu einer Lösung von 1,32 g (12 mMol) CaCl₂ in 10 ml Wasser zugetropft, die entstandene Suspension auf 0°C abgekühlt und scharf abgesaugt. Calcium-di-[4-(2,6-dichlorphenylimino)]-tetrahydro-3-thiophenacetat, das als Rückstand verbleibt, wird getrocknet und ohne weitere Reinigung weiterverarbeitet.

## Patentansprüche

1. Verbindungen der Formel I, 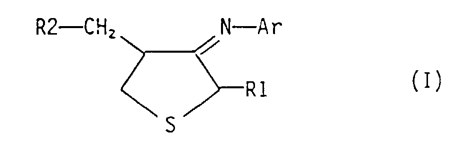 worin
R1 Wasserstoff oder -CO-O-R6 bedeutet,
R2 -CO-O-R7 bedeutet,
Ar 2,6-Dichlorphenyl bedeutet,
R6 Wasserstoff, Methyl oder Ethyl bedeutet und
R7 Wasserstoff, Methyl, Ethyl oder 2-Hydroxy-ethoxy-ethyl bedeutet,
und ihre Salze.

2. Verwendung von Verbindungen der Formel I nach Anspruch 1, worin R1 Wasserstoff bedeutet, und ihren Salzen, zur Herstellung von Verbindungen der Formel IV, 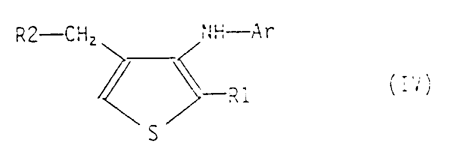 worin R1 Wasserstoff bedeutet und R2 und Ar die in Anspruch 1 genannten Bedeutungen haben, und ihren Salzen, dadurch gekennzeichnet, daß man Verbindungen der Formel I oder Ihre Salze mit Brom dehydriert und gewünschtenfalls anschließend erhaltene Salze in die freien Verbindungen oder erhaltene freie Verbindungen in die Salze überführt.

3. Verfahren zur Herstellung der Verbindungen der Formel I nach Anspruch 1 und ihrer Salze, dadurch gekennzeichnet, daß man Ketone der Formel V oder ihre Salze, 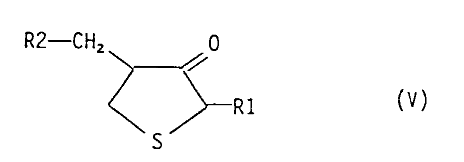 worin R1 und R2 die in Anspruch 1 angegebenen Bedeutungen haben, in Form ihrer Ketale mit 2,6-Dichloranilin umsetzt und gewünschtenfalls anschließend erhaltene Salze in die freien Verbindungen oder erhaltene freie Verbindungen in die Salze überführt.

## Claims

1. Compounds of the formula I, 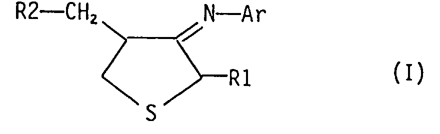 wherein
R1 denotes hydrogen or -CO-O-R6,
R2 denotes -CO-O-R7,
Ar denotes 2,6-dichlorophenyl,
R6 denotes hydrogen, methyl or ethyl and
R7 denotes hydrogen, methyl, ethyl or 2-hydroxy-ethoxy-ethyl,
and the salts of these compounds.

2. Use of compounds of the formula I according to Claim 1, wherein R1 denotes hydrogen, and their salts, for the preparation of compounds of the formula IV, 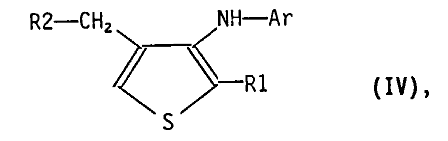 wherein R1 denotes hydrogen and R2 and Ar have the meanings given in Claim 1, and their salts, characterized in that compounds of the formula I or their salts are dehydrogenated with bromine and, if desired, salts obtained are then converted into the free compounds or free compounds obtained are converted into the salts.

3. Process for the preparation of the compounds of the formula I according to Claim 1 and their salts, characterized in that ketones of the formula V or their salts 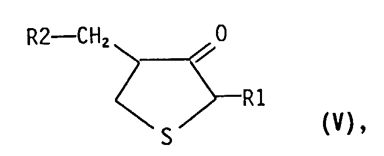 wherein R1 and R2 have the meanings mentioned in Claim 1, are reacted in the form of their ketals with 2,6-dichloroaniline and, if desired, salts obtained are then converted into the free compounds or free compounds obtained are converted into the salts.

## Revendications

1. Composés de formule I: 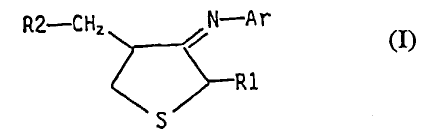 dans laquelle
R1 représente un atome d'hydrogène ou un groupe -CO-O-R6,
R2 représente un groupe -CO-O-R7,
Ar représente un groupe 2,6-dichlorophényle,
R6 représente un atome d'hydrogène ou un groupe méthyle ou éthyle, et
R7 représente un atome d'hydrogène ou un groupe méthyle, éthyle ou 2-hydroxy-éthoxy-éthyle,
et sels de tels composés.

2. Emploi de composés de formule I, conformes à la revendication 1, où R1 représente un atome d'hydrogène, ou de sels de tels composés, pour préparer des composés de formule IV: 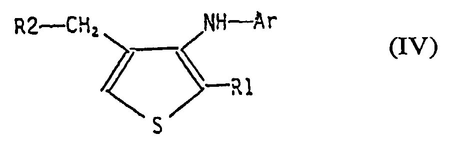 dans laquelle R1 représente un atome d'hydrogène et R2 et Ar ont les significations indiquées dans la revendication 1, ou des sels de ces composés, caractérisé en ce qu'on fait subir aux composés de formule I ou à leurs sels une déshydrogénation, à l'aide de brome, et l'on transforme ensuite, si on le souhaite, les sels ainsi obtenus en composés libres, ou bien les composés libres ainsi obtenus en sels.

3. Procédé de préparation de composés de formule I, conformes à la revendication 1, ou de sels de tels composés, caractérisé en ce que l'on fait réagir des cétones de formule V, ou leurs sels : 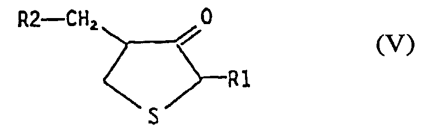 dans laquelle R1 et R2 ont les significations indiquées dans la revendication 1,
sous la forme de leurs cétals, avec de la 2,6-dichloroaniline, et l'on transforme ensuite, si on le souhaite, les sels ainsi obtenus en composés libres, ou bien les composés libres ainsi obtenus en sels.
